# EUROPEAN PATENT APPLICATION

(11) **EP 3 260 063 A1**
(43) Date of publication of application: **27.12.2017**
(21) Application number: 16175578.0
(22) Date of filing: 21.06.2016
(51) Int. Cl.: A61B 17/29, A61B 17/04

(54) **A SURGICAL DEVICE**

(71) Applicant: Topos Medical Limited, Dublin 18 (IE)
(72) Inventor: BRIDGES, Derek, Dublin, 18 (IE); LANG, Chris, Dublin, 18 (IE); KEANE, David, Dublin, 4 (IE); TOLAN, Michael, Dublin, 18 (IE)
(74) Representative: Purdy, Hugh Barry

(57) **Abstract**

A surgical device 1 made up of a hollow handle 2 having an elongate insertion portion 3 with a head 5 having jaws 6,7 at its free end, the head 5 being rotatable about a longitudinal axis 8 defined by the elongate insertion portion 3 and separately or simultaneously deflectable with respect to the insertion portion 3 at a joint 9 on the insertion portion 3, rotation and articulation of the head 5 and movement of the jaws 6,7 being effected via a head control mechanism 10 in the handle 2 made up of, firstly, a ring-like head rotation actuator 11, secondly, a separately and independently operable rotatable head deflection control 12 and, thirdly, a separately and independently operable lever-like jaw activator 13 at the handle 2.

## Description

### Introduction

This invention relates to a surgical device and more particularly to a surgical device having an effector end adapted to simultaneously rotate and deflect.

### Background of the Invention

Surgical devices are widely used for a range of operation types such as endoscopic and laparoscopic procedures. Generally, such surgical devices share a common structure and are made up of a handle and an elongate insertion portion for insertion in a body. The insertion portion is provided with an effector end which can be fitted with jaws for holding surgical instruments such as needles or can be adapted for cutting and sealing operations as required.

The effector end of known surgical devices is typically adapted to move so that a surgeon can perform operations with dexterity e.g. to axially rotate about the longitudinal axis of the insertion portion and/or deflect or hinge/articulate with respect to the insertion portion. Where the effector end is provided with jaws for holding surgical instruments, the jaws can also be opened and closed. Rotation, deflection and opening/closing of the jaws (or the performing of cutting/sealing operations and the like) is usually controlled by a surgeon via the handle.

Movement of the effector end of the surgical devices can be effected using various mechanical and/or motorised control mechanisms. However, a number of problems exist with such arrangements in known surgical devices. For example, where known surgical devices have an effector end which is adapted to rotate and deflect, the rotational and deflection movements of the effector end are generally mechanically coupled with the result that rotation of the effector end is limited by the degree to which the effector end is deflected. Accordingly, such surgical devices can prove difficult to use accurately in operations where a high degree of precision is required e.g. in extremely confined or difficult to access areas of the body.

Motorised surgical devices can assist in allowing rotational movement of the effector end. However, motorised surgical devices are not widely accepted by surgeons due to the lack of control or "feel" feedback sensed by surgeons in use. Moreover, as most surgical devices are used only once, the significant additional costs associated with motorised surgical devices can make their use economically untenable for many surgical procedures.

### Summary of the Invention

According to the invention there is provided a surgical device comprising:
a handle;
an elongate insertion portion attached to the handle;
a deflectable head with jaws on the elongate insertion portion;
a head control mechanism in the handle having a deflection control portion for controlling deflection of the head and a jaw activator portion for controlling opening/closing of the jaws wherein
the head control mechanism comprises a de-coupling structure for de-coupling deflection of the effector end and opening/closing of the jaws.

Preferably, the de-coupling structure comprises a carriage for the jaw activator portion, the carriage being movable in response to movement of the deflection control portion to maintain a constant relationship between the jaw activator portion and the jaws.

More preferably, the deflection control portion comprises a movable shaft in the insertion portion extending between the head and the handle. Most preferably, the shaft is attached to the carriage so that the carriage is moved in response to movement of the shaft.

Suitably, the shaft is attached to a deflector arm towards the head to effect deflection of the head via the arm.

Preferably, the deflector arm comprises an elbow to increase the angle of deflection.

Advantageously, the deflection control portion comprises a deflection gear train in the handle.

Preferably, the deflection gear train is attached to the shaft in the handle.

Advantageously, the deflection control portion further comprises a dial on the handle for actuating the deflection gear train.

Preferably, the jaw activator portion comprises a jaw cable extending between the jaws and the carriage. More preferably, the jaw cable is attached to a movable slider in the carriage.

Suitably, the slider is translationally movable by a lever on the handle to effect translational movement of the jaw cable.

Preferably, the surgical device further comprises a depressible button between the lever and the slider.

In a further embodiment, the head is rotatable about its longitudinal axis under the control of a head rotation actuator portion of the head control mechanism.

Preferably, the head rotation actuator portion comprises a head rotation cable extending between head and the handle.

Suitably, the head rotation cable comprises an endless cable movable between a rotatable spindle at the head and a rotatable input shaft at the handle.

Preferably, the head rotation actuator portion comprises a head rotation gearing at the handle for effecting movement of the rotatable input shaft and the spindle.

More preferably, the head rotation actuator portion further comprises a manually operable head rotation ring on the handle for actuating the head rotation gearing.

The head of the surgical device of the invention can be separately and independently manipulated by deflection and opening/closing of the jaws at the head i.e. the movements are de-coupled through the use of a head control mechanism having a de-coupling component. More particularly, the head control mechanism has a jaw activator portion which compensates for deflection of the head so that the jaws remain open/closed as required during deflection operations. Accordingly, a surgeon is free to deflect the head as required without being concerned with inadvertent movement of the jaws. As a result, the head can be safely deflected to a high deflection angle and used in confined and difficult spaces during surgery.

The angle to which the head can be deflected is also enhanced through the use of a deflector arm provided with an elbow at the head of the surgical device.

The head of the surgical device of the invention can be fully rotated by a jaw rotator cable extending between a spindle at the head and the handle. More particularly, precise and accurate manual full rotation of the head is achieved through translational movement of the cable between the spindle and the handle which results rotational movement of the spindle and the head. The rotational movement of the spindle allows for 360° rotation of the head.

The invention also relates to a method of performing a surgical procedure comprising a step of inserting at least a distal part of the surgical apparatus of the invention into a patient, and actuating the device to perform a surgical procedure. In one embodiment, the step of inserting the distal part of the surgical procedure is performed by means of keyhole surgery.

### Brief Description of the Drawings

The invention will now be described, by way of example only, with reference to the accompanying drawings in which:

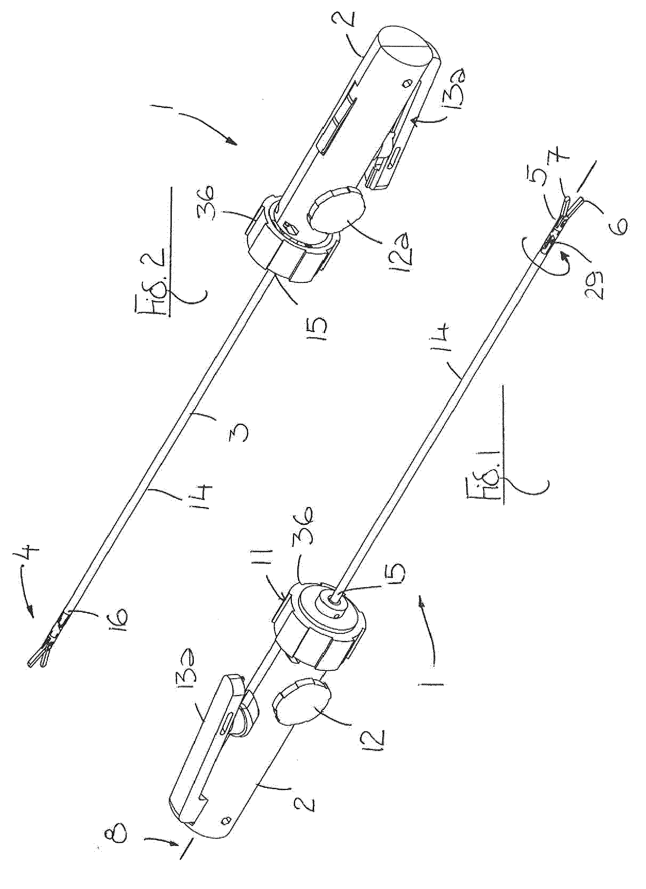

Figure 1 is a perspective view from above and one side of a surgical device of the invention having an effector end or head adapted to simultaneously and/or independently rotate about a longitudinal axis in the direction indicated by the arrow and deflect relative to the insertion portion of the surgical device with the jaws of the surgical device in the open position;
Figure 2 is a perspective view from the opposite end and side of the surgical device of Figure 1;

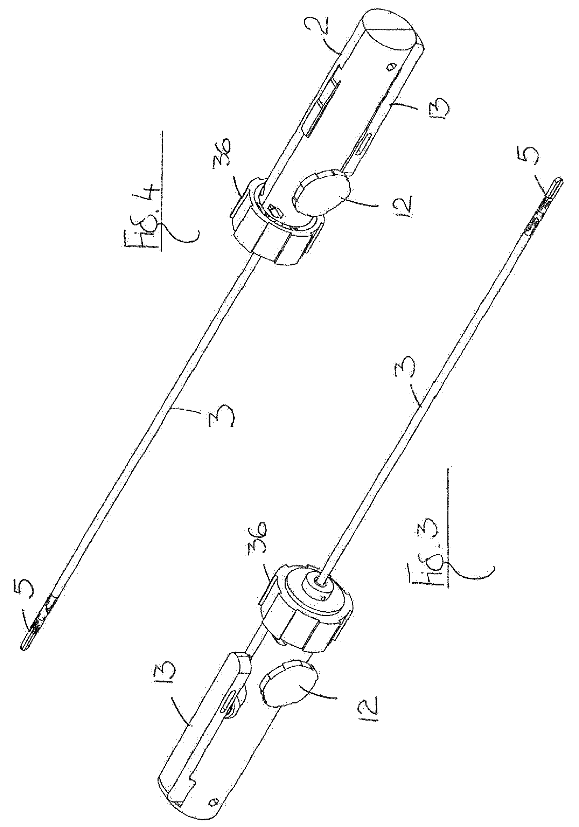

Figure 3 is a perspective view from above and one side of the surgical device with the jaws closed;
Figure 4 is a perspective view the opposite end and side of the surgical device of Figure 3;

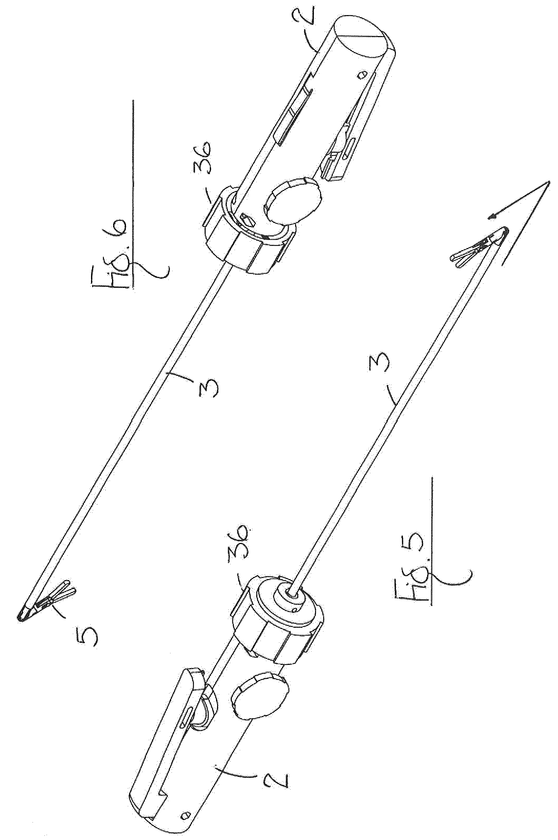

Figure 5 is a perspective view from above and one side of the surgical device with the jaws open and the head deflected;
Figure 6 is a perspective view from the opposite end and side of the surgical device of Figure 5;

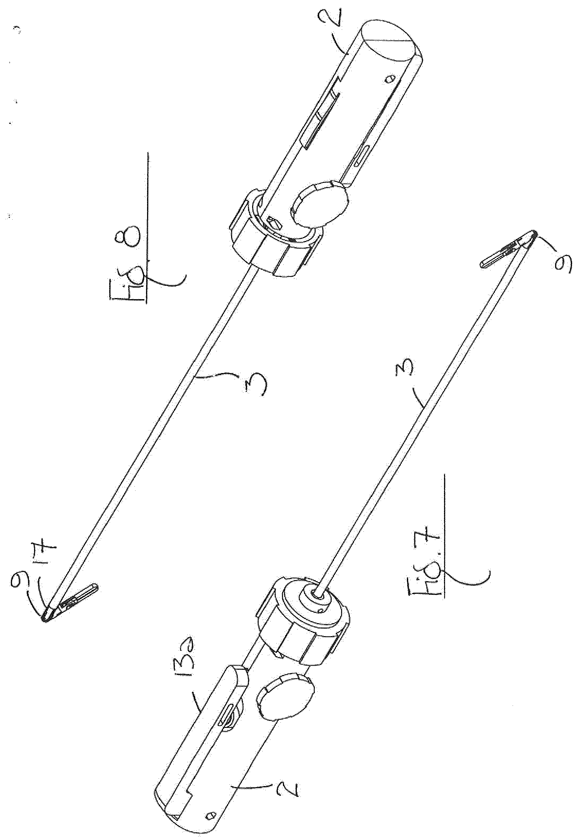

Figure 7 is a perspective view from above and one side of the surgical device with the jaws closed and the head deflected;
Figure 8 is a perspective view from the opposite end and side the surgical device of Figure 7;
Figure 9 is a side elevation of the surgical device of Figure 1;
Figure 10 is a cross-sectional view through the surgical device of Figure 9 showing the cable for effecting rotation of the head extending from the handle of the surgical device through the insertion portion to the head;
Figure 11 is an enlarged perspective cross-sectional view through the handle at the head rotation actuator;
Figure 12 is an enlarged perspective view from above and one side of the head of the surgical device of Figure 9 with the jaws open;
Figure 13 is a partially cutaway view of the head of Figure 12 with the head housing partially removed to show the neck spindle and the cable for effecting rotation of the head attached to the spindle;
Figure 14 is an enlarged perspective view from above and one side of the head of the surgical device with the head housing fully removed to more clearly show looping of the cable for effecting rotation of the head attached to the spindle;
Figure 15 is a side elevation of the surgical device similar to that of Figure 9 but with the head rotated trough 90°;
Figure 16 is a cross-sectional view through the surgical device of Figure 15 similar to that of Figure 10;
Figure 17 is an enlarged perspective view from above and one side of the head of the surgical device of Figure 15;
Figure 18 is a partially cutaway view of the head of Figure 17 similar to that of Figure 13;
Figure 19 is an enlarged perspective view from above and one side of the head of the surgical device of Figure 18 similar to that of Figure 14;
Figure 20 is a side elevation of the surgical device with the jaws in the open position and the head in an undeflected position;
Figure 21 is a cross-sectional view through the device of Figure 20 with the head rotation actuator portion and rotator cable of the head control mechanism removed to more clearly illustrate the deflection control portion of the head control mechanism;
Figure 22 is an enlarged perspective cross-sectional view of the deflection control portion of the head control mechanism of Figure 21;
Figure 23 is an enlarged perspective cross-sectional view of the undeflected head and distal end of the insertion portion of the device of Figure 21;
Figure 24 is a side elevation of the surgical device of Figure 20 with the jaws in the open position and the head in a deflected position;
Figure 25 is a cross-sectional view through the device of Figure 24 with the head rotation actuator portion of the head control mechanism removed to more clearly illustrate the deflection control portion of the head control mechanism with the toothed ring of the deflection control displaced by the external dial to effect deflection of the head ;
Figure 26 is an enlarged perspective cross-sectional view of the deflection control portion of the head control mechanism of Figure 25;
Figure 27 is an enlarged perspective cross-sectional view of the deflected head and distal end of the insertion portion of the device of Figure 25;
Figure 28 is a side elevation of the surgical device with the jaw activator portion of the head control mechanism in the jaw open position i.e. with the jaw lever in the raised position;
Figure 29 is cross-sectional view through the surgical device of Figure 28;
Figure 30 is an enlarged perspective cross-sectional view of the handle of the device of Figure 28 showing the slider of the jaw activator in the jaw open position;
Figure 31 is an enlarged perspective cross-sectional view of the head and distal end of the insertion portion with the jaws in the open position;
Figure 32 is a side elevation of the surgical device of Figure 28 with the jaw activator portion of the head control mechanism in the jaw closed position i.e. with the jaw lever in the depressed position;
Figure 33 is cross-sectional view through the surgical device of Figure 32;
Figure 34 is an enlarged perspective cross-sectional view of the handle of the device of Figure 32 showing the slider of the jaw activator in the jaw closed position;
Figure 35 is an enlarged perspective cross-sectional view of the head and distal end of the insertion portion with the jaws in the closed position;
Figure 36 is a cross-sectional view of the surgical device broadly similar to the surgical device as shown in Figure 29;
Figure 37 is an enlarged cross-sectional view through the surgical device of Figure 36;
Figure 38 is an enlarged partial cross-sectional view through the jaws, head and distal end of the insertion portion of the surgical device of Figure 36;
Figure 39 is an alternate enlarged partial cross-sectional view through the jaws, head and distal end of the insertion portion of the surgical device of Figure 36 showing the path of the jaw cable of the jaw activator through the neck spindle of the rotatable neck to the jaws;
Figure 40 is a cross-sectional view of the surgical device broadly similar to the surgical device as shown in Figure 33 with the jaws in the closed position;
Figure 41 is an enlarged cross-sectional view through the surgical device of Figure 40;
Figure 42 is an enlarged partial cross-sectional view through the jaws, head and distal end of the insertion portion of the surgical device of Figure 40, and
Figure 43 is an alternate enlarged partial cross-sectional view through the jaws, head and distal end of the insertion portion of the surgical device of Figure 40 showing the path of the jaw cable of the jaw activator through the neck spindle of the rotatable neck to the jaws.

### Detailed Description of the Invention

As shown in the drawings, a surgical device in accordance with the invention is generally indicated by the reference numeral 1 and is made up of a hollow handle 2 having an elongate insertion portion 3 extending therefrom provided with an effector end 4 in the form of a head 5 at its free end. The head 5 is made up of co-operable jaws 6,7 for holding a needle or similar implement during surgery and is rotatable about a longitudinal axis 8 defined by the elongate insertion portion 3 and separately or simultaneously deflectable or angularly orientable with respect to the insertion portion 3 at a joint 9 on the insertion portion 3 in the directions indicated by the arrows. Rotation and articulation of the head 5 and movement of the jaws 6,7 is effected using a head control mechanism 10 in the handle 2 made up of, firstly, a ring-like head rotation actuator 11 portion, secondly, a separately and independently operable rotatable head deflection control portion 12 and, thirdly, a separately and independently operable lever-like jaw activator portion 13 at the handle 2.

The insertion portion 3 is formed from a tube-like outer housing 14 having a handle proximal end 15 and a handle distal or free end 16 on which a head mounting 17 for supporting the head 5 is fixedly mounted. The head mounting 17 is hingedly attached to a head housing 18 of the head 5 at the joint 9 while the head housing 18 in turn abuts a rotatable neck 19 on which the jaws 6,7 of the head 5 are mounted so that the rotatable neck 19 can rotate in and with respect to the head housing 18 as described further below.

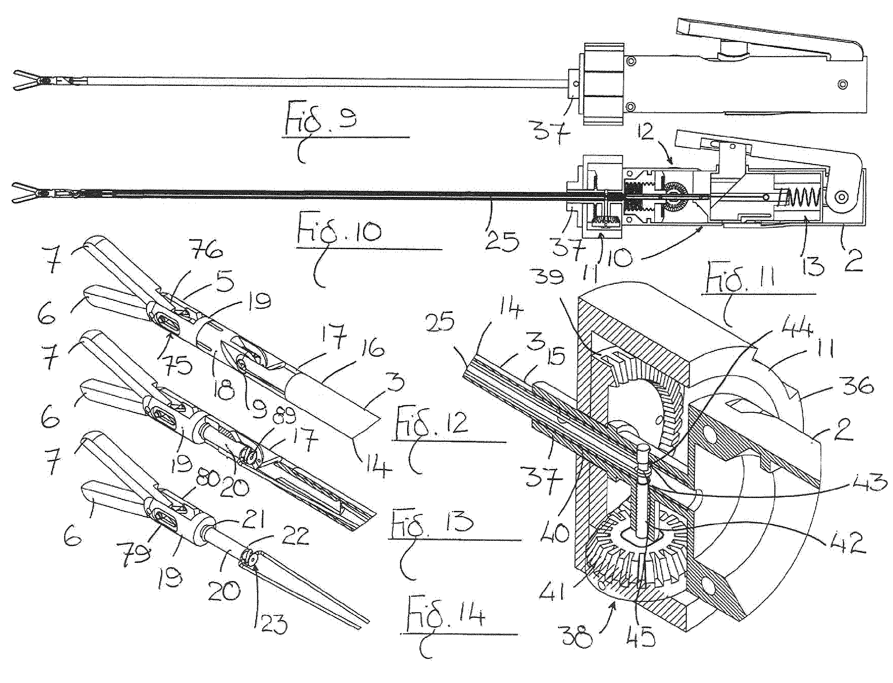

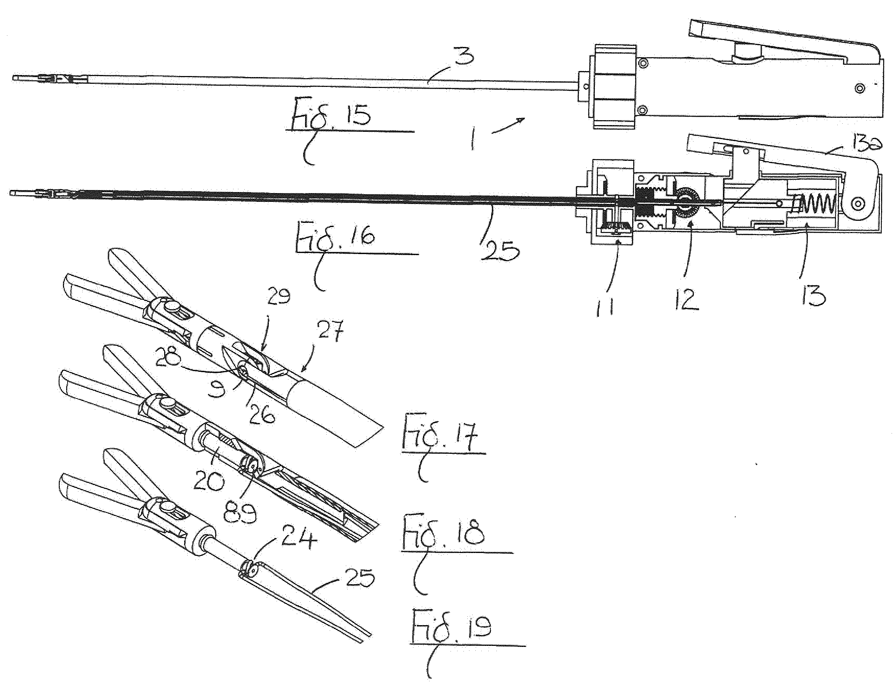

More particularly, as shown especially in Figures 9 to 19, the neck 19 is provided with a rearwardly extending cable-driven shaft-like spindle 20, extending towards the handle 2, having a neck end 21 attached to the neck 19 and a head mounting end 22 disposed towards and received in the head mounting 17 at its opposite free end. The spindle 20 is provided with a cable coupling 23 in the form of a cable receiving circumferential groove 24 at the head mounting end 22 for frictionally engaging an endless head rotator cable 25 for effecting rotation about the longitudinal axis 8.

The cable 25 is wound about the circumferential groove 24 of the spindle 20 and extends bilaterally from the groove 24 through the head mounting 17 into oppositely disposed first and second elongate lateral cable slots 26,27 respectively defined in the head mounting 17 at the joint 9 for guiding the rotator cable 25 from the head mounting 17 towards the handle 2 and the head rotation actuator 11 of the head control mechanism 10. More particularly, each cable slot 26,27 is provided with respective first and second cable holes 28,29 adjacent and either side of the circumferential groove 24 for receiving the rotator cable 25.

As shown particularly in Figure 11, the head rotation actuator 11 component of the head control mechanism 10 is made up of a manually operable head rotation ring 36 circumferentially mounted on the handle 2 adjacent a bushing 37 which extends into the handle 2 on the tube-like housing 14 of the insertion portion 3 and a head rotation gearing 38, mechanically attached to the external head rotation ring 36, contained within the handle 2 for effecting movement of the rotator cable 25 between the neck spindle 20 and the head rotation actuator 11.

The gearing 38 is made up of an input or drive gear 39 fixedly attached to and rotatable by the head rotation ring 36 about the bushing 37 and an internal portion 40 of the tube-like outer housing 14 of the insertion portion 3 which extends into the handle 2 at the handle proximal end 15 of the outer housing 14. The drive gear 39 is in turn engaged with an output or driven gear 41 mounted in a plane perpendicular to the drive gear 39 on a shaft 42 disposed perpendicular to the insertion portion 3 (and the longitudinal axis 8) and which extends through the bushing 37 and the internal portion 40 of the tube-like housing 14 at shaft openings 43 defined in the bushing 37 and the internal portion 40 of the tube-like housing 14.

In an analogous manner to the spindle 20, the shaft 42 is provided with a shaft cable mounting 44 in the form of a shaft cable groove 45 for receiving the rotator cable 25 in a wound manner about the groove 45.

Accordingly, rotation of the head 5 by a user is effected by manually rotating the head rotation ring 36 to in turn rotate the drive gear 39 so that the endless rotator cable 25 wrapped about the shaft groove 45 on the shaft 42 is rotated. Accordingly, oppositely disposed and spaced apart strands of the cable 25 are caused to translationally move in opposite directions so that the rotator cable 25 which is frictionally engaged with the spindle 20 effects rotation of the spindle 20 about the longitudinal axis 8 resulting in rotation of the neck 19 and jaws 6,7.

The neck 19 and jaws 6,7 of the head 5 can therefore be rotated through 360° as required from the handle 2.

As indicated above, the head 5 of the surgical device 1 can also be deflected with respect to the longitudinal axis 8 of the insertion portion 3. As shall be explained more fully below, a high degree of deflection can be achieved separately and independently of rotation of the head 5.

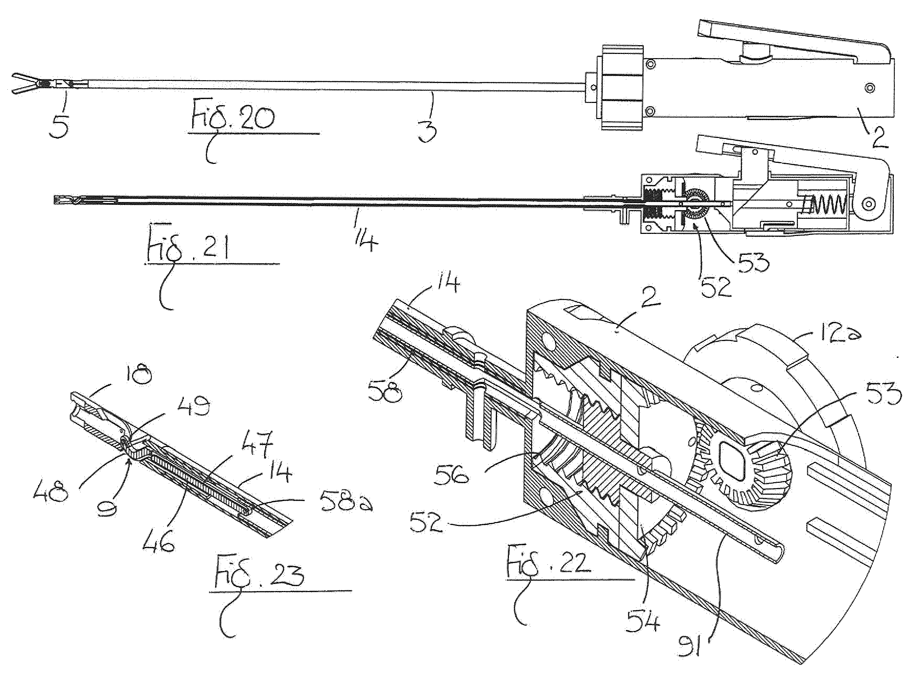

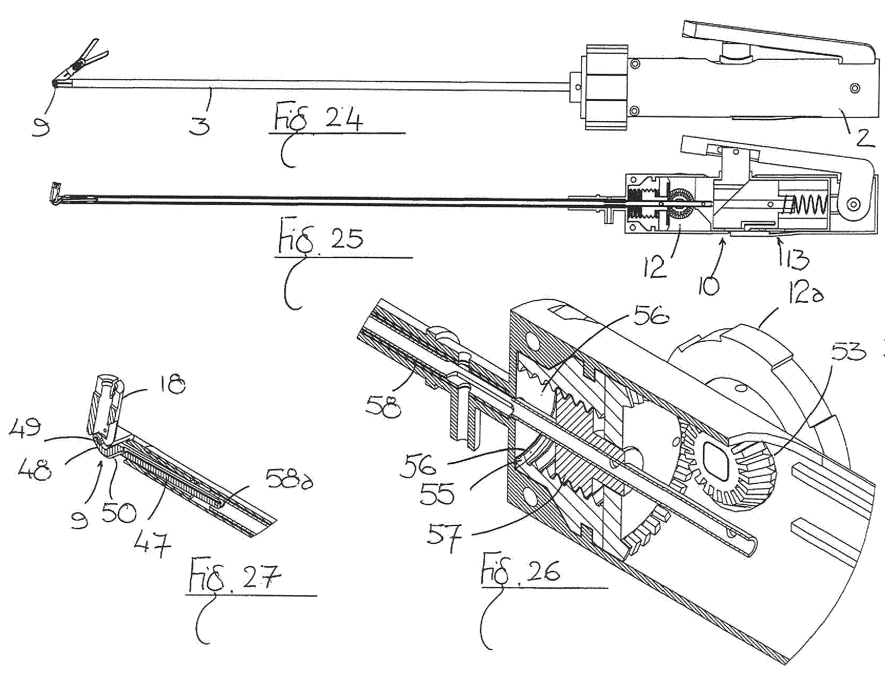

As shown particularly in Figures 20 to 26, deflection of the head 5 about the joint 9 is effected by a head deflection arm 46 slidably mounted in the outer tube-like housing 14 at the handle distal end 16 of the insertion portion 3.

The deflection arm 46 is made up of a straight or linear portion 47 parallel with the outer housing 14 and an elbow 48 disposed towards the head housing 18. The deflection arm 46 is attached to the head housing 18 at a deflection arm mounting 49 adjacent the hinge 9.

As shall be explained more fully below, the deflection arm 46 is provided with a jaw cable channel 50 for receiving a jaw cable 51 for effecting opening and closing of the jaws 6,7 under the control of the jaw activator portion 13 of the head control mechanism 10.

The deflection control 12 of the head control mechanism 10 in the handle 2 for controlling deflection of the head 5 about the joint 9 is made up of a dial-controlled deflection gear train 52 housed in the handle 2. The deflection gear train 52 is formed from a deflection drive gear 53 internally mounted within the handle 2 and connected to an external deflection dial 12a so that rotation of the deflection dial 12a drives the drive gear 53. The gear train 52 further includes a driven gear 54, mounted perpendicularly to and rotatably engaged with the drive gear 53. The driven gear 54 is fixedly attached to an annular drive gear 55 defining an internal threaded bore 56 extending about the longitudinal axis 8 so that the annular drive gear 55 is rotated by the driven gear 54. The annular drive gear 55 is in turn threadedly engaged with an externally threaded driven ring 57 within the threaded bore 56 so that rotation of the annular drive gear 55 results in translational movement of driven ring 57 in the threaded bore 56.

The driven ring 57 is in turn fixedly mounted on an elongate inner tubular shaft 58 contained within and contiguous with the outer housing 14 so that the translational movement of the driven ring 57 results in translational movement of the tubular shaft 58. The tubular shaft 58 extends from the handle 2 to the deflection arm 46 and is attached to the deflection arm 46 at a shaft mounting 58a.

Accordingly, manual rotation of the articulation dial 12a on the handle 2 results in actuation of the deflection gear train 52 to effect translational movement of the driven ring 57 via the annular drive gear 55 to effect movement of the shaft 58 which in turn causes the deflection arm 46 to deflect the head 5 about the hinge 9 as required.

As indicated above the jaws 6,7 can be opened and closed as required separately and independently of rotation and/or deflection of the head 5 via a jaw activator portion 13 of the head control mechanism 10.

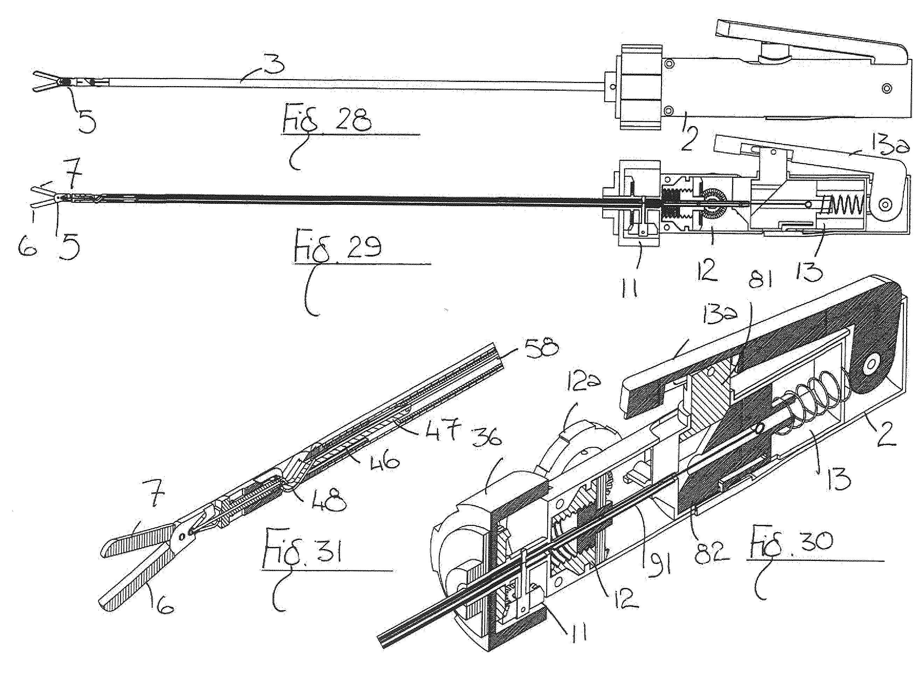

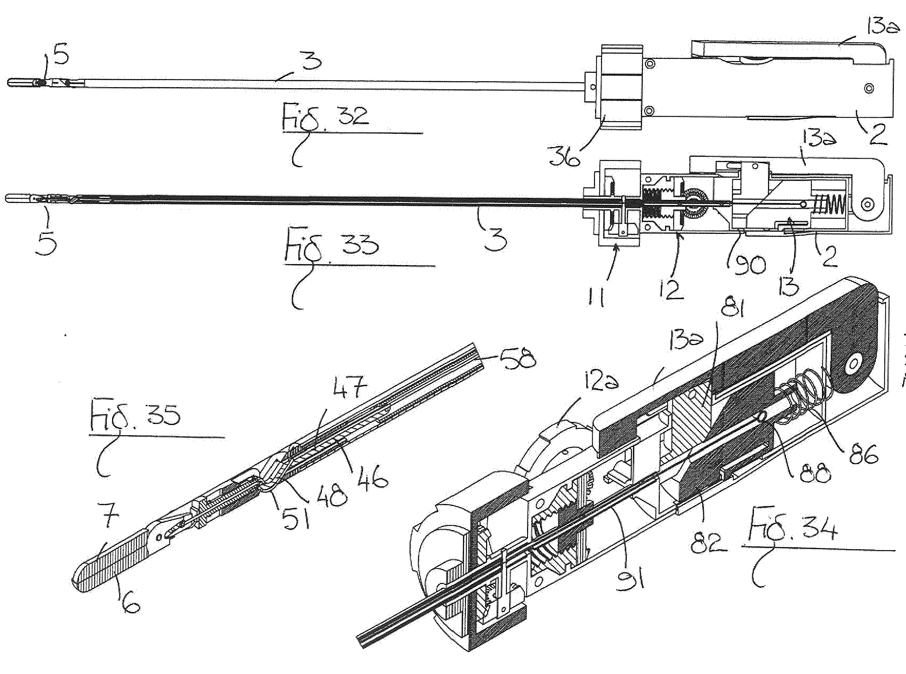

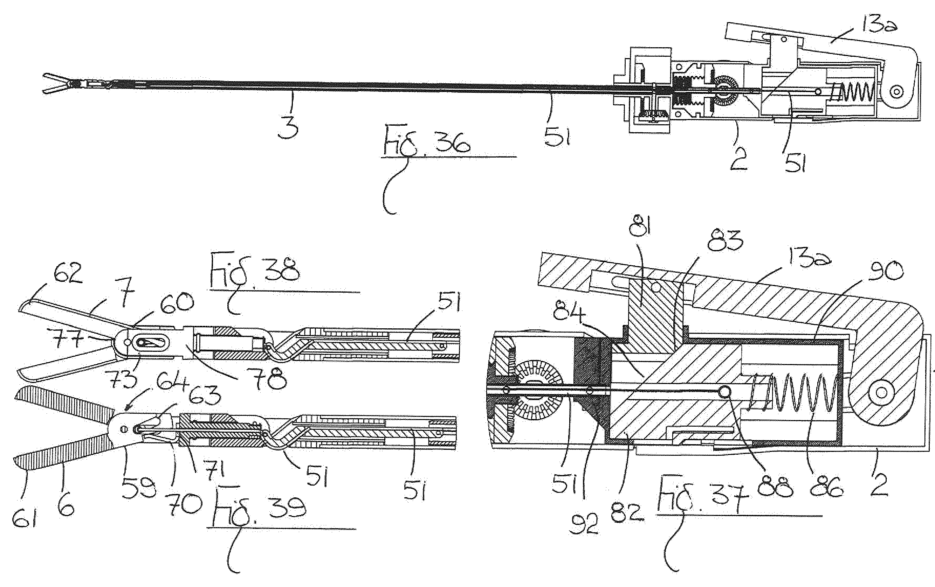

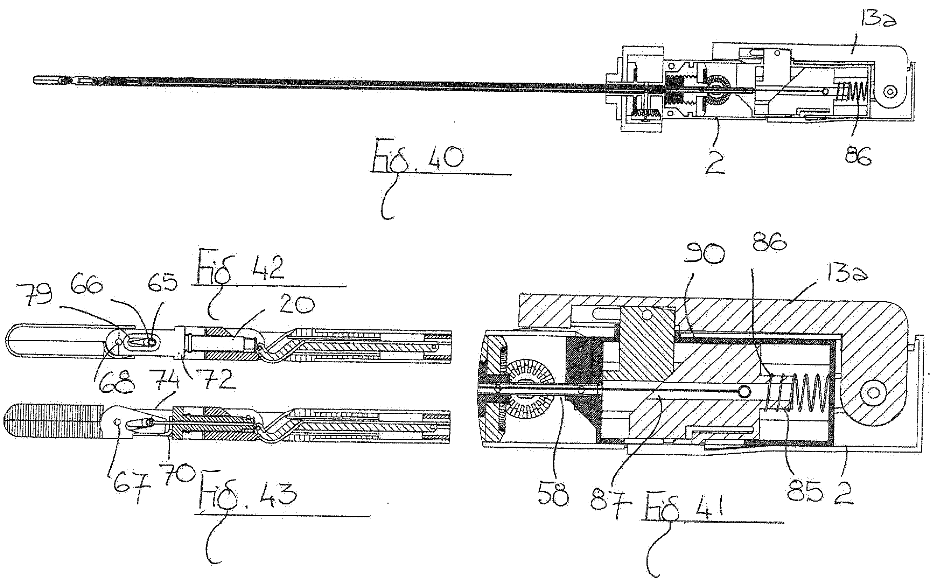

As shown particularly in Figures 28 to 41, the oppositely disposed jaws 6,7 are each made up of a hinged end 59,60 about which the jaws 6,7 can articulate and an open end 61,62 for holding implements such as needles as required. The jaws 6,7 are each provided at their hinged ends 59,60 with an elongate pin slot 63,64 for receiving a sliding transverse jaw pin 65 between the pin slots 63,64 to hingedly join jaws 6,7. The sliding jaw pin 65 is fitted with an end cap 66 at each end to hold the jaw pin 65 in the pin slots 63,64.

A fixed pin 67 also extends between the jaws 6,7 in a fixed pin hole 68 provided in each jaw 6,7 adjacent the pin slots 63,64.

The sliding jaw pin 65 is provided with a centrally located cable attachment slot 69 while the sliding jaw pin 65 is also mounted in a spring 70. The spring 70 is located between the jaws 6,7 and is biased so that, as discussed further below, upon actuation of the jaw activator 13, the jaws 6,7 are closed by the spring 70 via the jaw cable 51.

The spring 70 is provided with a cable through hole 71 to allow the jaw cable 51 to pass through the spring 70 to the sliding jaw pin 65.

The jaws 6,7, the sliding jaw pin 65 and the spring 70 are mounted and supported in the rotatable neck 19. More particularly, the rotatable neck 19 is substantially cylindrical in shape having a base 72 from which the spindle 20 projects towards the head mounting 17 and two spaced apart oppositely disposed fingers 73,74 upstanding from the base 72 towards the jaws 6,7. The fingers 73,74 are separated by oppositely disposed slits 75,76 and terminate at an open mouth 77 from which the jaws 6,7 project. Accordingly, the rotatable neck 19 defines an internal housing 78 for holding the jaws 6,7, the sliding jaw pin 65 and the spring 70. Each finger 73,74 is provided with an elongate moving pin slot 79,80 respectively for receiving the sliding jaw pin end caps 66. The end caps 66 therefore hold the jaws 6,7 in the rotatable neck 19 while the sliding jaw pin 65, and hence the jaws 6,7, can ride along the moving pin slots 79,80.

Opening and closing of the jaws 6,7 can be manually effected from the handle 2 via the jaw activator portion 13 of the head control mechanism 10 in the handle 2 which is in communication with the jaws 6,7 via the jaw cable 51. More particularly, the jaw activator 13 is made up of a hinged lever 13a on the handle 2 which is in mechanical communication with the jaws 6,7 via the jaw cable 51 which is attached to the cable attachment slot 69 on the sliding jaw pin 65 as described above.

The jaw activator 13 is also made up of a spring-biased depressible button 81 which protrudes upwards from the handle 2 beneath the lever 13a. The button 81 is in turn attached to a jaw slider block 82 which is horizontally slidably movable along the horizontal axis 8 in the handle 2 upon depression of the button 81 by the lever 13a. The depressible button 81 is substantially cylindrical in shape and has an inclined face 83 which abuts a complimentary similarly inclined face 84 on the slider block 82 so that vertical depression of the button 81 by the lever 13a results in horizontal translational movement of the slider block 82.

The slider block 82 is provided with a rearwardly extending shoulder 85 for receiving a spring 86 to bias the slider block 82 and in turn the button 81 against the lever 13a. Internally, the slider block 82 is provided with a horizontal cable channel 87 for receiving the jaw cable 51. The jaw cable 51 is fixed to the slider block 82 within the cable channel 87 at a jaw cable fixing 88 and extends from the jaw cable fixing 88 to the sliding jaw pin 65 through the tube like housing 14. The jaw cable 51 is guided to the neck 19 via the jaw cable channel 50 on the head deflection arm 46 which extends along the outer edge of the deflection arm 46 including the elbow 48.

The jaw cable 51 then extends from the elbow 48 into a jaw cable bore 89 defined along the central longitudinal axis of the neck spindle 20 and emerges from the jaw cable bore 89 to engage with the sliding jaw pin 65 as previously described.

In order to effect closure of the jaws 6,7, the lever 13a is depressed to urge the button 81 downwards against the biased sliding block 82 thereby translationally moving the sliding block 82 against the spring 86 to retract the sliding block 82 and the jaw cable 51 fixed to the sliding block 82 at the cable fixture 88 and then pull on the sliding jaw pin 65 to close the jaws 6,7.

As shall be explained more fully below, the sliding block 82 and the associated spring 86 are housed in a jaw actuator carriage 90 which is itself slidably mounted in the handle 2. In order to allow sliding movement of the carriage 90 in the handle 2 when required, the lever 13a is provided with a button duct 91 in which the button 81 is translationally slidable upon sliding of the carriage 90.

The carriage 90 is critical to allowing separate and independent rotation and deflection of the head 5 and opening and closing of the jaws 6,7. In particular, the carriage 90 is fixed to the shaft 58 described above for effecting deflection of the head 5 at a shaft extension 91 which extends rearwardly from the ring 57 of the deflection control portion 12 of the head control mechanism 10 to the carriage 90 and is secured to the carriage 90 at a shaft mounting 92 disposed towards the ring 57. Accordingly, translational movement of the shaft 58 of the deflection control 12 of the head control mechanism 10 results in a corresponding translational movement of the carriage 90 so that the length and tension of the jaw control cable 58 remains unaltered so that undesired movement of the jaws 6,7 does not occur i.e. the length of the jaw cable 58 as defined between the jaw cable fixing 88 and the head 5 remains unchanged as the carriage 90 housing the jaw activator 13 moves with the shaft 58 - i.e a constant relationship is maintained between the jaw activator 13 and the jaws 6,7.

Accordingly, in use, the head control mechanism 10 can be used to manipulate the effector end 4 of the surgical device by independent and/or simultaneous rotation, deflection and jaw opening/closing. The carriage 90 serves as a decoupling structure to prevent undesired opening/closing of the jaws 6,7 during deflection of the head 5.

The surgical device 1 of the invention employs two cables 25,50 to effect rotation of the head 5 and opening/closing movement of the jaws 6,7. The cables 25,50 can be discrete cables, single or multi strand either woven or plaited as desired. Suitable cables are formed from medical grade materials such as medical grade ultra high molecular weight polyethylene fibre. A preferred cable material is Dyneema Purity (Trade Mark) SGX fibre available from DSM Biomedical Incorporated. Such cables exhibit, *inter alia,* high tensile strength, high pliability, cut resistance, a low friction coefficient and are non-hemolytic.

## Claims

1. A surgical device comprising:
a handle;
an elongate insertion portion attached to the handle;
a deflectable head with jaws on the elongate insertion portion;
a head control mechanism in the handle having a deflection control portion for controlling deflection of the head and a jaw activator portion for controlling opening/closing of the jaws wherein
the head control mechanism comprises a de-coupling structure for de-coupling deflection of the head and opening/closing of the jaws.

2. A surgical device as claimed in Claim 1 wherein the de-coupling structure comprises a carriage for the jaw activator portion, the carriage being movable in response to movement of the deflection control portion to maintain a constant relationship between the jaw activator portion and the jaws.

3. A surgical device as claimed in Claim 2 wherein the deflection control portion comprises a movable shaft in the insertion portion extending between the head and the handle.

4. A surgical device as claimed in Claim 3 wherein the shaft is attached to the carriage so that the carriage is moved in response to movement of the shaft.

5. A surgical device as claimed in Claim 4 wherein the shaft is attached to a deflector arm towards the head to effect deflection of the head via the arm.

6. A surgical device as claimed in Claim 5 wherein the deflector arm comprises an elbow to increase the angle of deflection.

7. A surgical device as claimed in any of claims 3 to 6 wherein the deflection control portion comprises a deflection gear train in the handle.

8. A surgical device as claimed in Claim 7 wherein the deflection gear train is attached to the shaft in the handle.

9. A surgical device as claimed in Claim 7 or Claim 8 wherein the deflection control portion further comprises a dial on the handle for actuating the deflection gear train.

10. A surgical device as claimed in any of Claims 2 to 9 wherein the jaw activator portion comprises a jaw cable extending between the jaws and the carriage.

11. A surgical device as claimed in Claim 10 wherein the jaw cable is attached to a movable slider in the carriage.

12. A surgical device as claimed in Claim 11 wherein the slider is translationally movable by a lever on the handle to effect translational movement of the jaw cable.

13. A surgical device as claimed in Claim 12 further comprising a depressible button between the lever and the slider.

14. A surgical device as claimed in any of Claims 1 to 13 wherein the head is rotatable about its longitudinal axis under the control of a head rotation actuator portion of the head control mechanism, wherein the head rotation actuator portion optionally comprises a head rotation cable extending between head and the handle.

15. A surgical device as claimed in Claim 15 wherein the head rotation cable comprises an endless cable movable between a rotatable spindle at the head and a rotatable input shaft at the handle, wherein the head rotation actuator portion optionally comprises a head rotation gearing at the handle for effecting movement of the rotatable input shaft and the spindle, wherein the head rotation actuator portion optionally further comprises a manually operable head rotation ring on the handle.
